# EUROPÄISCHE PATENTANMELDUNG

(11) **EP 0 536 549 A1**
(43) Veröffentlichungstag der Anmeldung: **14.04.1993**
(21) Anmeldenummer: 92115406.8
(22) Anmeldetag: 09.09.1992
(51) Int. Cl.: A61B 17/34

(54) **Trokarhülse zum Durchführen eines medizinischen Instrumentes**

(30) Priorität: 09.10.1991 DE 9112550 U
(71) Anmelder: Richard Wolf GmbH, D-75438 Knittlingen (DE)
(72) Erfinder: Hiltebrandt, Siegfried, W-7134 Knittlingen (DE)
(74) Vertreter: Vollmann, Heiko, Dipl.-Ing.

(57) **Zusammenfassung**

Es ist eine Trokarhülse (1) zum Durchführen eines medizinischen Instrumentes, beispielsweise eines Endoskopes, beschrieben. Sie umfaßt einen Hohlschaft (2) und eine daran proximalseitig befestigte, einen Axialdurchgang (5) aufweisende Handhabe (4) mit einer Dichtungseinrichtung (10) für eine Abdichtung des Axialdurchgangs (5) und des Hohlschaftes (2) sowohl bei eingeführtem Instrument als auch bei nichteingeführtem Instrument. Sie kennzeichnet sich dadurch, daß die Dichtungseinrichtung (10) einen ein- oder mehrteiligen Trennwandaufbau (11,12) aus elektrisch nichtleitendem, elastischem Material aufweist, der derart durchbrochen (13) ist, daß er bei nichteingeführtem Instrument zur Abdichtung des Hülsendurchgangs (3,5) eine geschlossene, abdichtende Wand darstellt.

## Beschreibung

Die Erfindung geht aus von einer Trokarhülse zum Durchführen eines medizinischen Instrumentes, beispielsweise eines Endoskopes, mit einem Hohlschaft und einer daran proximalseitig befestigten, einen Axialdurchgang aufweisenden Handhabe mit einer Dichtungseinrichtung für eine Abdichtung des Axialdurchgangs und des Hohlschaftes sowohl bei eingeführtem Instrument als auch bei nichteingeführtem Instrument.

Eine derartige Trokarhülse ist z. B. in den DE-Gebrauchsmustern 70 04 051, 74 30 345 und 77 35 963 beschrieben. Bei diesen Trokarhülsen umfaßt die Dichtungseinrichtung eine distale Dichtungseinheit aus einer Ventileinrichtung, die eine vollständige Abdichtung des Axialdurchgangs der Handhabe bewirkt, wenn das medizinische Instrument, z. B. ein Trokar oder ein Endoskop, aus der Trokarhülse herausgezogen worden ist. Die betreffenden Ventileinrichtungen bestehen aus Ventilen der Klappen- oder Kugelbauart. Bei diesen vorbekannten Trokarhülsen kann es vorkommen, daß bei Verwendung von stromführenden medizinischen Instrumenten, z. B. eines mit HF-Strom versorgten Operationsinstrumentes, ein Stromübergang durch die Trokarhülse hindurch auf das diese Hülse umgebende Körpergewebe des Patienten durch die Ventilmetallteile übertragen werden kann. Des weiteren behindern die distalen Ventile durch ihre Ventilkörper die Handhabung der eingeführten Instrumente doch recht erheblich und sind nach Gebrauch der Trokarhülse nicht leicht vollständig zu reinigen. Insofern hat man Trokarhülsen entwickelt, deren Handhabe anstelle eines Ventils einen drehbaren Scheibenteil aufweist, der achsparallel zum Schaft der Trokarhülse verdrehbar ist und neben einer Abdichtfläche einen freien Instrumentendurchgang hat (US-PS 41 12 932). Dieser Instrumentendurchgang wird zum Schaftkanal der Trokarhülse in Überdeckung gebracht, so daß dann das gewünschte Instrument eingeführt werden kann. Auch hier besteht die Gefahr des Stromübergangs, und ferner bedeutet das Vorsehen des Scheibenteils eine zusätzliche Bedienungstätigkeit und eine vergrößerte Bauweise der Trokarhülse sowie höhere Herstellungskosten.

Die Aufgabe der Erfindung besteht darin, eine Trokarhülse der einleitend angefürten Art so zu verbessern, daß ein Stromübergang auf das an der Hülse anliegende Körpergewebe des Patienten sicher vermieden, eine leichte und vollständige Reinigung der Trokarhülse auch in ihrem Abdichtungsbereich ermöglicht, eine sichere Abdichtung sowohl bei eingeführtem als auch bei entferntem Instrument gegenüber dem körperinneren Überdruck eines insufflierten Gases gewährleistet und die Handhabung eines eingeführten Instrumentes nicht behindert.

Die Lösung dieser Aufgabe ist in dem Anspruch 1 angeführt.

Eine bevorzugte und einfache Ausführungsform dieser Lösung besteht darin, daß der Trennwandaufbau aus zwei Dichtungsscheiben mit zentraler, normal geschlossener und vorzugsweise als Kreuzschlitzung ausgeführter Schlitzung besteht, wobei die beiden geschlitzten Dichtungsscheiben aufeinanderliegend in der Handhabe umfangsmäßig eingespannt sind.

Mit dieser Lösung ist auf einfache Weise eine Trokarhülse geschaffen, bei der sowohl bei eingeführtem als auch bei herausgezogenem bzw. nichteingeführtem Instrument eine sichere Abdichtung geschaffen ist und bei der ein Stromübergang auf das an die Trokarhülse angrenzende Körpergewebe des Patienten sicher vermieden ist, denn ein stromleitendendes Teil weist der abdichtende Trennwandaufbau nicht auf. Dieser Aufbau ist ferner sehr kompakt und verursacht nur geringe Herstellungskosten und läßt sich wegen seiner einfachen Gestalt sehr gut reinigen und desinfizieren. Des weiteren wird bei eingeführtem Instrument eine sichere Gasabdichtung gegenüber einem körperinneren, auf einer Gasinsufflation beruhenden Überdruck bis etwa 65 Hektopascal und höher erreicht. Auch gewährleistet die erfindungsgemäße distale Abdichtungseinheit eine sehr gute Handhabung eines eingeführten Instrumentes, d. h. das Instrument ist durch die Abdichtungsrichtung praktisch nicht behindert. Schließlich ist auch eine Wartung oder Reparatur der Trokarhülse einfach durchzuführen, weil die scheibenförmige Dichtungsrichtung schnell gegen eine neue ausgetauscht werden kann, indem ein Handhabenteil nur abgeschraubt und nach Auswechselung von Dichtungsscheiben durch einfaches Herausnehmen und Einsetzen von Scheiben wieder aufgeschraubt zu werden braucht.

In bevorzugter Weitergestaltung der erfindungsgemäßen Dichtungseinrichtung ist eine gute Abdichtung bei in die Trokarhülse eingeführtem Instrument dadurch erreicht, daß beispielsweise eine zusätzliche Dichtungsscheibe mit zentralem, das eingeführte Instrument abdichtendem Loch vorgesehen ist, die an der oder den geschlitzten Dichtungsscheiben axial anliegt oder davon beabstandet ist. Alternativ kann auch so vorgegangen sein, daß ein einstückiges Dichtungsteil vorgesehen ist, das in axialer Ausrichtung sowohl die geschlitzte, normal geschlossenwandige Dichtungsausbildung als auch das erwähnte zentrale Loch aufweist.

Die Erfindung ist nachstehend anhand mehrerer, in der anliegenden Zeichnung dargestellter Ausführungsbeispiele näher erläutert. Es zeigen:
- Figur 1: einen überwiegenden Axialschnitt durch ein erstes Ausführungsbeispiel,
- Figur 2: eine Abdichtungsteil dafür in Aufsicht,
- Figur 3: ein weiteres Ausführungsbeispiel, teilweise im Axialschnitt,
- Figuren 4 und 5: Abdichtungsteile dafür in Aufsicht bzw. im Schnitt,
- Figur 6: ein drittes, teilweise gezeigtes Ausführungsbeispiels im Axialschnitt.

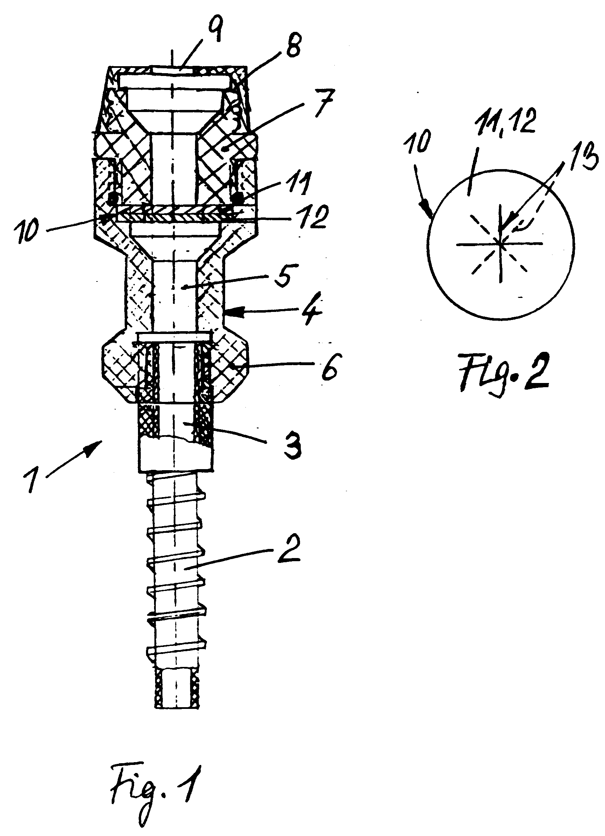

Gemäß Figur 1 besteht die allgemein mit 1 bezeichnete Trokarhülse aus einem üblichen Schaft 2 mit innerem Durchführungskanal 3 und einer proximal am Schaft 2 befestigten Handhabe 4 mit ebenfalls einem zentralen Axialdurchgang 5. Die Handhabe 4 selbst besteht aus einem ersten Teil 6, das mit dem Schaft 2 verschraubt ist, und aus einem zweiten Teil 7 als Einführungsstutzen, das mit dem ersten Teil 6 verschraubt ist.

Die beiden Teile 6 und 7 der Handhabe 4 bestehen entweder aus elektrisch nichtleitendem Material, z. B. aus Kunststoff, oder sind in herkömmlicher Weise aus Metall gefertigt und mit einem elektrisch nichtleitenden Material innen ausgekleidet. Ebenfalls ist auch der Schaft 2 insgesamt oder durch eine Auskleidung aus Kunststoff elektrisch nichtleitend.

Das zweite Teil 7 der Handhabe 4 ist proximalseitig mit einer üblichen elastischen Dichtungskappe 8 mit einer üblichen zentralen Durchbrechung 9 versehen. Die Durchbrechung 9 umschließt den Schaft eines in die Trokarhülse 1 eingeführten medizinischen Instrumentes zwecks Abdichtung gegenüber einem durch Insufflation in der betreffenden Körperhöhle des Patienten erzeugten Überdruckes.

Um aber auch eine Abdichtung bei in den Körper des Patienten eingeführter Trokarhülse 1 zu erreichen, wenn sich kein Instrument in der Trokarhülse befindet, ist die Handhabe etwa in ihrer Längsmitte mit einem Trennwandaufbau 10 mit Dichtungsfunktion versehen. Dieser Trennwandaufbau besteht aus elektrisch nichtleitendem, elastischem Material und ist mittig derart durchbrochen, daß er bei nicht eingeführtem Instrument eine geschlossene, abdichtende Wand darstellt.

Der normal geschlossenwandige Trennwandaufbau 10 besteht vorzugsweise aus zwei aufeinanderliegenden, kreisförmigen, geschlitzten Scheiben 11,12, die umfangsmäßig zwischen den Teilen 6 und 7 der Handhabe 4 eingespannt sind. Bei gewissen Anwendungsfällen kann es ausreichen, daß nur eine Dichtungsscheibe vorgesehen ist.

Aus Figur 2 ist die Schlitzung der Dichtungsscheiben 11,12 eindeutig zu erkennen. Diese zentrale, normal, d.h. bei nichteingeführtem Instrument, geschlossene Schlitzung 13 besteht vorzugsweise aus einer Kreuzschlitzung. Entsprechend den Anwendungsfällen kann auch eine andere Schlitzung gewählt werden. Bei Verwendung von zwei oder mehreren Dichtungsscheiben 11,12 können diese so in der Handhabe 4 eingesetzt sein, daß die gewählten Schlitzungen jeweils zweier einander benachbarter Scheiben gegeneinander versetzt angeordnet sind. In Figur 2 ist dies durch die gestrichelte Darstellung der Schlitzung 13 der unteren Dichtungsscheibe 12 beispielsweise gezeigt. Voraussetzung ist in jedem Fall, daß die Schlitzung bei nicht eingeführtem Instrument eine ausreichende Dichtung gegenüber dem erwähnten Gasüberdruck ergibt. Ein bevorzugtes Material für die elastischen Dichtungsscheiben besteht aus Polyetherketon, welches Material ein thermoplastischer Kunststoff ist.

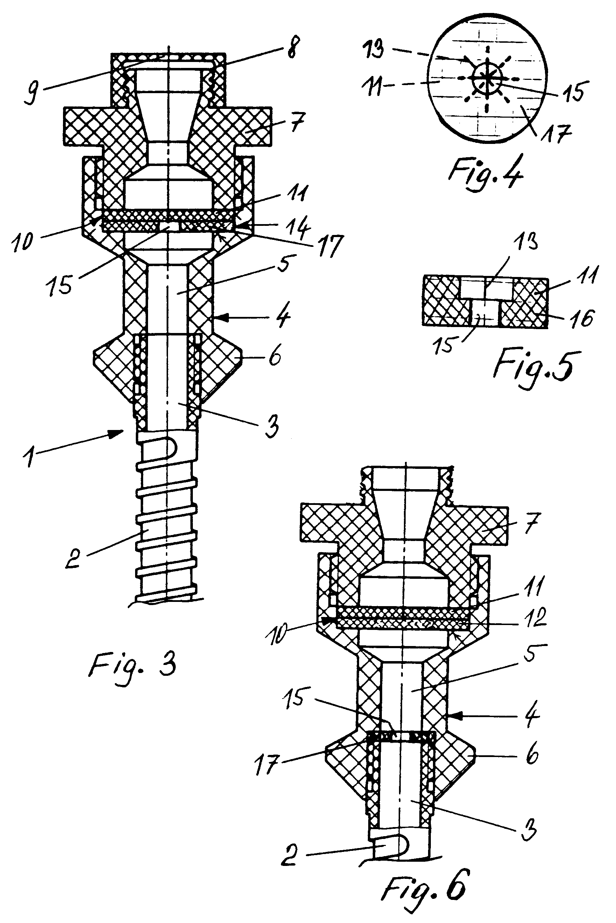

Das Ausführungsbeispiel nach Figur 3 unterscheidet sich dadurch von demjenigen nach Figur 1, daß der geschlitzte Trennwandaufbau 10, der hier nur aus einer einzigen geschlitzten Dichtungsscheibe 11 besteht, aber auch hier aus mehreren solchen Scheiben bestehen kann, zusätzlich mit einem Dichtungsaufbau 14 mit einem zentralen Loch 15 aus elastischem, elektrisch nichtleitendem Material kombiniert ist. Dieser Dichtungsaufbau kann in Form wenigstens einer zusätzlichen Scheibe 17, wie es in den Figuren 3 und 4 gezeigt ist, aber auch in Form eines gelochten Teilbereiches 16 vorgesehen sein, der zusammen mit der geschlitzten Scheibe 11 des Trennwandaufbaus 10 ein einstückiges Bauteil bildet, wie Figur 5 zeigt.

In dem Fall, wo mehrere gelochte Scheiben 17 vorgesehen sind, können sie beidseitig der geschlitzten Scheibe 11 oder Scheiben 11,12 anliegen. Auch ist es möglich, zusätzlich oder alternativ wenigstens eine gelochte Scheibe mit axialem Abstand zu der oder den geschlitzten Scheiben 11,12 vorzusehen. Letzteres zeigt Figur 6, wo eine zentral gelochte Scheibe 17 z.B. zwischen dem Handhabenteil 6 und dem Hohlschaft 2 eingespannt ist.

Wenn es gewünscht wird, kann bei den Beispielen nach den Figuren 3 bis 6 noch zusätzlich die bekannte Dichtungskappe 8 vorgesehen sein, wie Figur 3 zeigt.

Obwohl es bevorzugt wird, für den zusätzlichen Dichtungsaufbau 14 ebene Lochscheiben vorzusehen, können auch hiervon abweichende Dichtungselemente verwendet werden, die ähnlich oder vergleichbar geformt sind, z.B. membranartig oder kegelstumpfartig, so daß auch diese Formen vom Schutz miterfaßt sein sollen. Ein wichtiges Merkmal des Dichtungsaufbaus besteht auch darin, daß er in bezug auf den Trennwandaufbau 10 eine neue Position einnimmt.

## Patentansprüche

1. Trokarhülse zum Durchführen eines medizinischen Instrumentes, beispielsweise eines Endoskopes, mit einem Hohlschaft und einer daran proximalseitig befestigten, einen Axialdurchgang aufweisenden Handhabe mit einer Dichtungseinrichtung für eine Abdichtung des Axialdurchgangs und des Hohlschaftes sowohl bei eingeführtem Instrument als auch bei nichteingeführtem Instrument, dadurch gekennzeichnet, daß die Dichtungseinrichtung einen ein- oder mehrteiligen Trennwandaufbau (10) aus elektrisch nichtleitendem, elastischem Material aufweist, der derart durchbrochen (13) ist, daß er bei nichteingeführtem Instrument zur Abdichtung des Hülsendurchgangs (3,5) eine geschlossene, abdichtende Wand darstellt.

2. Trokarhülse nach Anspruch 1, dadurch gekennzeichnet, daß der Trennwandaufbau (10) aus wenigstens einer Dichtungsscheibe mit zentraler Schlitzung (13) besteht.

3. Trokarhülse nach Anspruch 2, dadurch gekennzeichnet, daß zwei oder mehrere geschlitzte Dichtungsscheiben (11, 12) aufeinanderliegend vorgesehen und in der Handhabe (4) umfangsmäßig eingespannt sind.

4. Trokarhülse nach Anspruch 2 oder 3, dadurch gekennzeichnet, daß die Schlitzung (13) der Dichtungsscheibe (11) oder der Dichtungsscheiben (11, 12) aus einer Kreuzschlitzung besteht.

5. Trokarhülse nach Anspruch 2,3 oder 4, dadurch gekennzeichnet, daß die Schlitzungen (13) einander benachbarter Dichtungsscheiben (11,12) gegeneinander versetzt angeordnet sind.

6. Trokarhülse nach Anspruch 1, dadurch gekennzeichnet, daß der Trennwandaufbau (10) mit einem zusätzlichen, zentral gelochten Dichtungsaufbau (14) aus elastischem, elektrisch nichtleitendem Material in Form wenigstens eines zentral gelochten, scheibenartigen Teils (17), das an der oder den geschlitzten Dichtungsscheiben (11,12) anliegt oder bzw. auch davon axial beabstandet ist, oder in Form eines mit ihm einstückig ausgebildeten Teilbereiches (16) kombiniert ist.

7. Trokarhülse nach Anspruch 6, dadurch gekennzeichnet, daß ein zentral gelochtes, scheibenartiges Teil (17) distalseitig der geschlitzten Scheibe (11) oder Scheiben (11,12) zwischen der Handhabe (4) und dem Hülsenhohlschaft (2) eingespannt ist.

8. Trokarhülse nach Anspruch 1, dadurch gekennzeichnet, daß das Material des Trennwandaufbaus (10) aus Polyetherketon besteht.
